(19) Europäisches Patentamt
European Patent Office
Office européen des brevets

(11) EP 1 618 877 A1

(12) **EUROPEAN PATENT APPLICATION**
published in accordance with Art. 158(3) EPC

(43) Date of publication:
**25.01.2006 Bulletin 2006/04**

(21) Application number: **04728895.6**

(22) Date of filing: **22.04.2004**

(51) Int Cl.:
*A61K 31/155* (1985.01) *A61P 31/02* (2000.01)
*A61P 31/04* (2000.01) *A01N 25/02* (1985.01)
*A01N 25/22* (1985.01) *A01N 25/30* (1985.01)
*A01N 47/44* (1985.01) *A61L 2/18* (1980.01)

(86) International application number:
**PCT/JP2004/005776**

(87) International publication number:
**WO 2004/093859 (04.11.2004 Gazette 2004/45)**

(84) Designated Contracting States:
**AT BE BG CH CY CZ DE DK EE ES FI FR GB GR HU IE IT LI LU MC NL PL PT RO SE SI SK TR**
Designated Extension States:
**AL HR LT LV MK**

(30) Priority: **22.04.2003 JP 2003011611**

(71) Applicant: **Dainippon Sumitomo Pharma Co., Ltd.**
**Osaka 541-8524 (JP)**

(72) Inventor: **SAITO, Koichi**
**Amagasaki-shi,**
**Hyogo 6610012 (JP)**

(74) Representative: **Vossius & Partner**
**Siebertstrasse 4**
**81675 München (DE)**

# (54) DILUTION-TYPE STERILIZER COMPOSITION

(57) The present invention provides a germicidal antiseptic composition for dilution, which is superior in preparation stability, and free of formation of insoluble precipitation even when diluted with water containing an inorganic ion (e.g., tap water etc) when in use, thereby retaining superior germicidal disinfection ability. The present invention relates to a germicidal antiseptic composition for dilution, which is an aqueous liquid comprising chlorhexidine gluconate as a main ingredient, (1) 1-10 w/v% of chlorhexidine gluconate; (2) 1-10 w/v% of one or more selected from the group consisting of a polyoxyethylene alkyl ether and a polyoxyethylene alkenyl ether, each having an HLB of 10-15 and a congeal point of not more than 35°C; (3) 0.001-0.5 w/v% of a water-soluble organic monocarboxylic acid having 2 to 6 carbon atoms; and (4) water.



Printed by Jouve, 75001 PARIS (FR)

## Description

### TECHNICAL FIELD

The present invention relates to a novel germicidal antiseptic. More particularly, the present invention relates to a novel germicidal antiseptic composition for dilution comprising chlorhexidine gluconate, which is

(1) superior in preparation stability, and
(2) free of formation of insoluble precipitation even when diluted with water containing an inorganic ion (e.g., tap water etc) when in use, thereby retaining superior germicidal antiseptic ability.

### BACKGROUND ART

Chlorhexidine gluconate is active against on a broad range of microorganisms and shows good antimicrobial ability even at a low concentration. In addition, chlorhexidine gluconate causes only little irritation in living organisms. Chlorhexidine gluconate has been used in the form of an aqueous solution or alcohol solution for disinfection of hands and skin, disinfection of operation site, disinfection of medical instruments, disinfection of wound site of the skin, disinfection of operation room, patient's room etc., and the like.
In general, germicidal antiseptics containing chlorhexidine gluconate are largely divided into two kinds of preparations depending on the purpose of use.
A first preparation is a liquid used for disinfection of skin, disinfection of operation site, disinfection of medical instruments, operation room and patient's room, disinfection of wounded skin, and the like.
When in use, the concentration of chlorhexidine gluconate in this liquid is adjusted depending on the subject and level of germicidal disinfection. In clinical situations, 5% to 20% aqueous chlorhexidine gluconate solution is generally diluted with water or alcohol as necessary before use. A preferable concentration range after dilution is 0.01-0.5 wt%.
A second preparation is directly used solely for disinfection of hands without dilution. Such preparation contains a high concentration surfactant or bubbling agent to add a cleansing effect. In addition, it may also contain a water-soluble polymer and the like to increase viscosity, thereby affording a sticking effect.
Furthermore, there are quick drying preparations to be thoroughly rubbed into hands. This type of preparation does not require rinsing away with water. This preparation has increased viscosity by the addition of a water-soluble polymer etc. and has quick drying property by containing alcohol at a high concentration.
These preparations are required to maintain antimicrobial ability and to show high stability as a pharmaceutical product. Particularly, in the case of a preparation to be diluted when in use, like the above-mentioned first preparation, the preparation is required to have not only stability before dilution but also stability after dilution.
Chlorhexidine has low solubility in water (0.08 w/v% at 20°C). Chlorhexidine becomes soluble in water upon conversion to gluconate (not less than 50 w/v% at 20°C). In addition, chlorhexidine has property to form a slightly water-soluble salt with a certain kind of anion. It is known that, when diluted with, for example, regular tap water, therefore, a chlorhexidine gluconate solution forms, a slightly water-soluble salt with various anions ($SO_4^{2-}$, $NO_3^-$, $Cl^-$ etc.) contained in tap water, which is precipitated with the lapse of time. When various inorganic metal ions ($Na^+$, $Mg^{2+}$, $Ca^{2+}$ etc.) are present, moreover, gluconic acid in a chlorhexidine. gluconate solution forms a salt with these ions, thereby easily causing an insoluble precipitation (Shokichi Furuhashi, Japan Medical Journal, No. 2734 (1976)).
The largest problem in precipitation is that insolubilization from chlorhexidine gluconate by various ions etc. in a preparation or diluted solution strikingly lowers the antimicrobial ability.
Therefore, a chlorhexidine gluconate-containing germicidal antiseptic composition to be used for dilution is considered to be desirably diluted with distilled water according to a pharmaceutical product package insert and the like. However, in actual situations, the composition is often diluted with tap water before use. Accordingly, a germicidal antiseptic composition for dilution containing chlorhexidine gluconate, which is free of precipitation of an insoluble material even when diluted with tap water containing various ions, is desired.
As a method of stabilizing chlorhexidine gluconate against anion, the following methods are known.

(1) JP-A-9-301858 describes a method of stably maintaining chlorhexidine gluconate in an aqueous preparation, even in the presence of a chlorine ion by adding a polycarboxylic acid having a particular structure or a salt thereof to an aqueous liquid containing 0.001-0.05 w/v% of chlorhexidine gluconate.
This discloses that, in an aqueous liquid such as eye drop and nose drop, wherein a chlorine ion is often contained in the composition, a time-course precipitation from chlorhexidine gluconate does not occur in the presence of a polycarboxylic acid (e.g., citric acid etc.) or a salt thereof, even in the presence of a chlorine ion. Generally, however, precipitation does not occur at a low concentration of not more than 0.01 w/v%, due to the solubility (0.06 w/v% at 20°C) of chlorhexidine hydrochloride and the like. In the case of an aqueous chlorhexidine gluconate preparation

prepared for dilution, since its concentration is generally as high as about 5 w/v%, addition of polycarboxylic acids, a salt thereof and the like causes precipitation in a preparation.

Moreover, tap water contains calcium ion, magnesium ion, potassium ion, sodium ion and the like corresponding to the total hardness of not more than 300 ppm. As anion in the amount corresponding to these metal ions, several dozens ppm or more each of chlorine ion, and sulfate ion and nitrate ion that promote precipitation from chlorhexidine gluconate is contained. Since these cause generation of insoluble materials from chlorhexidine gluconate, as a method for preventing precipitation when a chlorhexidine gluconate-containing aqueous preparation is diluted with tap water, the method of the above-mentioned JP-A-9-301858 is not sufficient.

(2) As a means for preventing precipitation of chlorhexidine due to various ions in a chlorhexidine gluconate-containing aqueous preparation, a method comprising adding a non-ionic surfactant, preferably nonylphenoxypoly(ethyleneoxy)ethanol, is known. However, nonylphenoxypoly(ethyleneoxy)ethanol is a major causative substance of endocrine disruptor-like substance. In view of the possibility of phase out in the future, the development of a substitute is desired. When a surfactant other than non-ionic surfactants, particularly an anionic or amphoteric surfactant, is used, a salt or a complex is formed with gluconic acid or chlorhexidine, like the above-mentioned inorganic ion.

Many germicidal antiseptics containing chlorhexidine gluconate and a non-ionic surfactant have been proposed heretofore.

For example, JP-B-6-31417 discloses an antibacterial cleaning agent comprising a chlorhexidine salt, a nonylphenoxypoly(ethyleneoxy)ethanol surfactant, polyethylene glycol fatty acid diester or fatty acid amide, polyethylene glycol ether of lanolin surfactant, and water.

JP-2961556 discloses a composition for skin disinfection, which contains a particular amount each of chlorhexidine gluconate, polyoxyethylene alkyl ether, fatty acid diethanolamide, alkyldimethylamine oxide and macrogol in an aqueous solvent.

However, since aim of these compositions is disinfection and washing of hands, the amount of the surfactant to be added is set for as high as 7-40 w/v% relative to 0.5-10 w/v% of chlorhexidine gluconate, and they cannot be used for disinfection for operation site and for wounded skin, or for germicidal disinfection of medical instruments. In addition, these preparations do not aim at preventing precipitation upon dilution with water containing various ions, such as tap water.

(3) JP-A-2000-273004 shows a germicidal antiseptic composition comprising a specific amount of polyalkylene glycol having a congeal point of not less than 35°C or a derivative of alkylene glycol having a congeal point of not less than 35°C, and a lower alcohol solution of a quaternary ammonium salt germicidal antiseptic or a biguanide germicidal antiseptic. This aims at reducing irritation to the skin and improving tactile sensation after use, such as roughness, stickiness etc. of the skin surface, by adding a particular substance to a lower alcohol solution of a quaternary ammonium salt germicidal antiseptic or a biguanide germicidal antiseptic. This does not aim at preventing precipitation associated with dilution with water containing various ions (e.g., tap water).

In general, germicidal antiseptic used for disinfection of operation site and wound site or germicidal disinfection of medical instruments is desirably free of components other than active ingredients for germicidal disinfection. Particularly, in the case of a germicidal antiseptic containing chlorhexidine gluconate as a main ingredient, the presence of a surfactant not only becomes a factor of reducing the germicidal disinfection ability of chlorhexidine gluconate but also tends to induce irritation to the application site. Therefore, the amount of addition thereof is desirably made as small as possible.

When the amount of a non-ionic surfactant is greater than necessary; it may incorporate chlorhexidine gluconate into a micelle and reduce its germicidal disinfection ability. However, when the amount of addition is reduced, the stabilizing effect becomes lower and the precipitation preventing effect is also lowered.

In the case of non-ionic surfactants held to be preferable for preventing precipitation, moreover, it is less known that the structure of lipophilic group or hydrophilic group produces difference in the stabilizing effect of chlorhexidine gluconate, and addition of a given amount may fail to sufficiently prevent precipitation.

Therefore, the development of a germicidal antiseptic composition for dilution, which contains small amounts of surfactant and alcohol, which does not allow insoluble precipitation of chlorhexidine when diluted with tap water, and which does not impair germicidal disinfection ability of chlorhexidine gluconate is required.

## Disclosure of the Invention

It is a purpose of the present invention to provide a germicidal antiseptic composition for dilution,

(1) which is superior in preparation stability, and

(2) which does not permit insoluble precipitation even when diluted with inorganic ion-containing water such as tap water etc. when in use, and can stably contain chlorhexidine gluconate in a solution, whereby superior germicidal

disinfection ability can be retained.

The present inventors have conducted intensive studies in an attempt to solve the aforementioned problems and found that, when a particular non-ionic surfactant and a particular organic acid are simultaneously contained in an aqueous chlorhexidine gluconate solution, superior preparation stability can be achieved and chlorhexidine gluconate can be maintained stably even when diluted with water containing various inorganic ions, such as tap water. They have conducted further studies and completed the present invention.

Accordingly, the present invention relates to:

[1] a germicidal antiseptic composition for dilution, which is an aqueous liquid comprising chlorhexidine gluconate as a main ingredient, comprising:

(1) 1-10 w/v% of chlorhexidine gluconate;
(2) 1-10 w/v% of one or more selected from the group consisting of a polyoxyethylene alkyl ether and a polyoxyethylene alkenyl ether, each having an HLB of 10-15 and a congeal point of not more than 35°C;
(3) 0.001-0.5 w/v% of a water-soluble organic monocarboxylic acid having 2 to 6 carbon atoms; and
(4) water;

[2] the germicidal antiseptic composition for dilution of the above-mentioned [1], wherein the water-soluble organic monocarboxylic acid having 2 to 6 carbon atoms is one or more selected from the group consisting of acetic acid, gluconic acid and gluconodeltalactone;
[3] the germicidal antiseptic composition for dilution of the above-mentioned [1] or [2], further comprising a water-soluble alcohol having 1 to 3 carbon atoms at not more than 10 w/v%;
[4] the germicidal antiseptic composition for dilution of any of the above-mentioned [1]-[3], wherein an alkyl chain of polyoxyethylene alkyl ether is an alkyl group having 10 to 14 carbon atoms, and an alkenyl chain of the polyoxyethylene alkenyl ether is an alkenyl group having 14 to 18 carbon atoms;
[5] the germicidal antiseptic composition for dilution of any of the above-mentioned [1]-[4], wherein the number of moles of ethylene oxide addition in polyoxyethylene alkyl ether is within the range of 7 to 20, and the number of moles of ethylene oxide addition in polyoxyethylene alkenyl ether is within the range of 7 to 20;
[6] the germicidal antiseptic composition for dilution of the above-mentioned [1], which has a chlorhexidine gluconate content within the range of 4-6 w/v%;
[7] the germicidal antiseptic composition for dilution of the above-mentioned [1], wherein the amount of one or more selected from the group consisting of polyoxyethylene alkyl ether and polyoxyethylene alkenyl ether, each having an HLB of 10-15 and a congeal point of not more than 35°C, is within the range of 2-7 w/v%;
[8] the germicidal antiseptic composition for dilution of the above-mentioned [1], wherein the amount of the water-soluble organic monocarboxylic acid having 2 to 6 carbon atoms is within the range of 0.01-0.2 w/v%;
[9] the germicidal antiseptic composition for dilution of any of the above-mentioned [1]-[8], wherein the polyoxyethylene alkenyl ether is polyoxyethylene oleyl ether;
[10] a germicidal antiseptic preparation to have a chlorhexidine gluconate content within the range of 0.05-0.5 w/v% by diluting the germicidal antiseptic composition for dilution of the above-mentioned [1] with water having a total hardness of not more than 300 mg/L or ethanol;
[11] a method of preventing precipitation of chlorhexidine gluconate under dilution with hard water, which comprises simultaneously adding a water-soluble organic monocarboxylic acid having 2 to 6 carbon atoms, and one or more selected from the group consisting of a polyoxyethylene alkyl ether having an HLB of 10-15 and a congeal point of not more than 35°C and a polyoxyethylene alkenyl ether having an HLB of 10-15 and a congeal point of not more than 35°C, to an aqueous liquid containing chlorhexidine gluconate as a main ingredient; and the like.

## DETAILED DESCRIPTION OF THE INVENTION

The present invention is explained in detail in the following.

The germicidal antiseptic composition for dilution of the present invention is an aqueous liquid containing chlorhexidine gluconate as an active ingredient.

Chlorhexidine gluconate is a compound represented by the following formula:

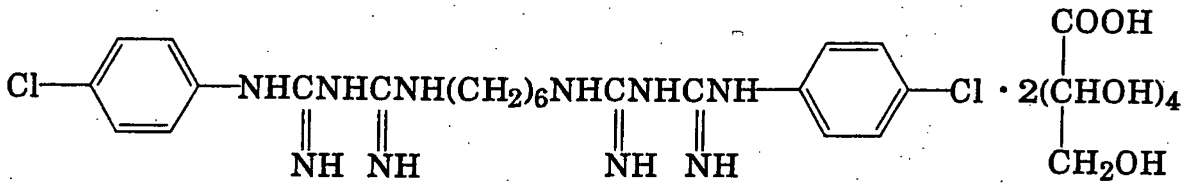

Chlorhexidine gluconate to be used in the present invention only needs to be pharmaceutically acceptable chlorhexidine digluconate. This is generally available as Japanese Pharmacopoeia chlorhexidine gluconate solution (aqueous solution containing 19-21 w/v% as chlorhexidine gluconate).

The germicidal antiseptic composition for dilution of the present invention is adjusted to contain chlorhexidine gluconate in a concentration of 1-10 w/v%, preferably 2-7 w/v%, more preferably 4-6 w/v%, so that dilution with water affords any concentration suitable for the purpose.

The second component to be contained in the germicidal antiseptic composition for dilution of the present invention is one or more selected from polyoxyethylene alkyl ether and polyoxyethylene alkenyl ether, each having an HLB of 10-15 and a congeal point of not more than 35°C.

Polyoxyethylene alkyl ether is represented by the formula:

$$H\text{-}(OCH_2CH_2)_a\text{-}O\text{-}C_mH_{2m+1}$$

and polyoxyethylene alkenyl ether is represented by the formula:

$$H\text{-}(OCH_2CH_2)_b\text{-}O\text{-}C_nH_{2n-1}$$

wherein a and b show the number of moles of ethylene oxide addition, and m and n are each an integer showing the carbon number of the alkyl chain and the alkenyl chain.

HLB shows a hydrophilic - lipophilic balance of a surfactant (W.C. Griffin, J. Soc. Cosmetic Chemists, 1, 311 (1949)). In this description, when the surfactant has a polyoxyethylene chain alone as a hydrophilic group, the HLB value can be determined by the formula:

$$HLB=E/5$$

wherein E is a weight fraction of an oxyethylene group (Tokiyuki Yoshida et al., new Surfactant Handbook, p 234, Kougakutosho (1987)).

When the second component in the germicidal antiseptic composition for dilution of the present invention shows an HLB exceeding 15, the hydrophilicity becomes too high, and the precipitation preventing effect of a surfactant on chlorhexidine gluconate tends to be reduced. Conversely; when it is less than 10, the lipophilicity becomes high and the precipitation preventing effect on chlorhexidine gluconate tends to be reduced because of its water solubility.

When the congeal point exceeds 35°C, crystallinity of the surfactant becomes high even if HLB is within the range of 10-15. As a result, the low temperature stability of the finally prepared aqueous liquid becomes reduced.

Polyoxyethylene alkyl ether having an HLB of 10-15 and a congeal point of not more than 35°C, which is the second component of the germicidal antiseptic composition for dilution of the present invention, is an adduct of various moles of ethylene oxide to saturated aliphatic alcohol. In addition, polyoxyethylene alkenyl ether having an HLB of 10-15 and a congeal point of not more than 35°C, which is the second component of the germicidal antiseptic composition for dilution of the present invention, is an adduct of various moles of ethylene oxide to unsaturated aliphatic alcohol. As the second component in the present invention, one or more pharmaceutically acceptable members can be selected from the group consisting of polyoxyethylene alkyl ether and polyoxyethylene alkenyl ether, each having an HLB of 10-15 and a congeal point of not more than 35°C.

Of these, the second component of the germicidal antiseptic composition for dilution of the present invention is preferably selected from, for example, polyoxyethylene alkyl ether, each having an alkyl chain having 10 to 14 carbon

atoms, and polyoxyethylene alkenyl ether having an alkenyl chain having 14 to 18 carbon atoms. As such polyoxyethylene alkyl ether, for example, polyoxyethylene decyl ether, polyoxyethylene undecyl ether, polyoxyethylene dodecyl ether (sometimes described as polyoxyethylene lauryl ether), polyoxyethylene tridecyl ether, and polyoxyethylene tetradecyl ether can be mentioned. As such polyoxyethylene alkenyl ether, for example, polyoxyethylene tetradecenyl ether, polyoxyethylene hexadecenyl ether, polyoxyethylene octadecenyl ether (e.g., polyoxyethylene oleyl ether etc.) and the like can be mentioned. The alkyl chain and alkenyl chain may be a straight chain or a branched chain. However, since the germicidal antiseptic composition for dilution of the present invention is applied to operation site and wound site on the skin surface besides medical instruments, the second component of the germicidal antiseptic composition for dilution of the present invention, one having a straight chain alkyl (or alkenyl) having an even number of carbon atoms is preferable. These have relatively high safety to living organisms.

When an alkyl chain of polyoxyethylene alkyl ether has more than 14 carbon atoms, the congeal point becomes higher, and a stabilizing effect on chlorhexidine gluconate tends to decrease. The same applies to an alkenyl chain of polyoxyethylene alkenyl ether having more than 18 carbon atoms. When an alkyl chain of polyoxyethylene alkyl ether has less than 10 carbon atoms, the precipitation preventing effect on chlorhexidine gluconate tends to be lower. The same applies to an alkenyl chain of polyoxyethylene alkenyl ether having less than 14 carbon atoms.

As the second component of the germicidal antiseptic composition for dilution of the present invention, of those having a straight chain alkyl (or alkenyl) having an even number of carbon atoms as mentioned above, one easily available and having shown actual performance as a pharmaceutical product is preferable from the economical aspect and the like. To be specific, polyoxyethylene lauryl ether and polyoxyethylene oleyl ether are preferably mentioned.

Generally, the congeal point of polyoxyethylene alkyl ether and polyoxyethylene alkenyl ether is determined by the length of alkyl (alkenyl) chain and the number of moles of ethylene oxide addition. Therefore, an ethylene oxide adduct having a congeal point of not more than 35°C is selected from the above-mentioned polyoxyethylene alkyl ether and polyoxyethylene alkenyl ether.

Of these, while the number of moles of ethylene oxide addition in polyoxyethylene alkyl ether varies depending on the length of the alkyl chain, when the length of the alkyl chain is 10-14, polyoxyethylene alkyl ether can be selected from those having the number of moles of ethylene oxide addition of 7-20, preferably 7-15. In addition, while the number of moles of ethylene oxide addition in polyoxyethylene alkenyl ether varies depending on the length of the alkenyl chain, when the length of the alkenyl chain is 14-18, polyoxyethylene alkenyl ether can be selected from those having the number of moles of ethylene oxide addition of 7-20.

When the number of moles of ethylene oxide addition in polyoxyethylene alkyl ether and polyoxyethylene alkenyl ether exceeds 20, not only the congeal point increases but also hydrophilicity becomes too high. As a result, a precipitation preventing effect on chlorhexidine gluconate tends to become low. Conversely, when it is less than 7, solubility in water becomes low, and preparation of a uniform aqueous solution tends to become difficult.

Preferable examples of the second component of the germicidal antiseptic composition for dilution of the present invention specifically include polyoxyethylene lauryl ether having the number of moles of ethylene oxide addition of 7-15 and polyoxyethylene oleyl ether having the number of moles of ethylene oxide addition of 7-20. Of these, more preferred are polyoxyethylene lauryl ether having the number of moles of ethylene oxide addition of 7-11 and polyoxyethylene oleyl ether having the number of moles of ethylene oxide addition of 7-15.

Of these, a particularly preferable second component of the germicidal antiseptic composition for dilution of the present invention is polyoxyethylene oleyl ether having the number of moles of ethylene oxide addition of 9-12. In the present invention, by selecting polyoxyethylene oleyl ether having a comparatively long chain alkyl group as the second component, a fine germicidal antiseptic composition for dilution superior not only in the stability against precipitation of chlorhexidine gluconate but also in the safety to the skin can be prepared.

It is known that HLB of surfactants can be adjusted to any value by mixing one having a low HLB and one having a high HLB. For the second component of the present invention, too, two or more can be selected from the group consisting of polyoxyethylene alkyl ether and polyoxyethylene alkenyl ether and mixed for use to achieve a final HLB of 10-15 and a congeal point of not more than 35°C.

The content of the second component of the germicidal antiseptic composition for dilution of the present invention is preferably selected from the range of 1-10 w/v%, more preferably the range of 2-7 w/v%, still more preferably the range of 3-5 w/v%, of the finally prepared germicidal antiseptic composition for dilution.

When the amount exceeds 10 w/v%, germicidal disinfection ability of chlorhexidine gluconate may be reduced on dilution. Conversely, when it is less than 1 w/v%, a sufficient precipitation preventing effect on chlorhexidine gluconate unpreferably cannot be achieved on dilution.

The third component of the germicidal antiseptic composition for dilution of the present invention is a water-soluble organic monocarboxylic acid having 2 to 6 carbon atoms. By adding this in combination with the above-mentioned second component, a precipitation preventing effect on chlorhexidine gluconate is remarkably improved.

As the third component of the germicidal antiseptic composition for dilution of the present invention, a pharmaceutically acceptable one can be appropriately selected from water-soluble organic monocarboxylic acid having 2 to

6 carbon atoms. In this description, "organic monocarboxylic acid" encompasses a dehydrate thereof, unless otherwise specified. As the water-soluble organic monocarboxylic acid having 2 to 6 carbon atoms, for example, acetic acid, propionic acid, lactic acid, gluconic acid, or gluconodeltalactone and the like can be specifically mentioned. Gluconodeltalactone is known to easily convert to gluconic acid in water. Of these, as a third component of the present invention, one or more kinds selected from the group consisting of acetic acid, gluconic acid and gluconodeltalactone can be preferably mentioned.

As a particularly preferable third component of the germicidal antiseptic composition for dilution of the present invention, gluconic acid and its dehydrate, gluconodeltalactone (sometimes to be described as glucono-δ-lactone), can be mentioned. By using these third components, a good germicidal antiseptic composition for dilution superior not only in the stability against precipitation of chlorhexidine gluconate but also in the safety to the skin can be prepared.

Here, while polycarboxylic acids such as citric acid, tartaric acid etc. are carboxylic acids commonly used as pH adjusting agents etc. of pharmaceutical products, addition thereof to the germicidal antiseptic composition for dilution of the present invention is not preferable because a precipitation preventing effect on chlorhexidine gluconate is low or it may conversely promote precipitation. Moreover, addition of metal salts of polycarboxylic acids such as citric acid, tartaric acid etc. (sodium salt, calcium salt, magnesium salt and the like) is not preferable, because it may promote precipitation from chlorhexidine gluconate.

The content of the water-soluble organic monocarboxylic acid having 2 to 6 carbon atoms, which is the third component of the germicidal antiseptic composition for dilution of the present invention, is selected from the range of 0.001-0.5 w/v%, more preferably the range of 0.01-0.2 w/v%, still more preferably the range of 0.02-0.1 w/v%.

When the amount of the third component is lower than 0.001 w/v%, a precipitation preventing effect on chlorhexidine gluconate tends to decrease. When it conversely exceeds 0.5 w/v%, the finally prepared germicidal antiseptic composition for dilution has low pH, which in turn may unpreferably promote decomposition of chlorhexidine gluconate and other components, and may increase skin irritation.

The germicidal antiseptic composition for dilution of the present invention is an aqueous solution wherein the aforementioned first to third components are dissolved in water, which is the fourth component.

As the fourth component of the germicidal antiseptic composition for dilution of the present invention, one may be appropriately selected from water generally used for pharmaceutical products, and specific examples thereof include water, purified water, water for injection and the like. The purified water here means water purified by ion exchange, ultrafiltration, distillation, or a combination thereof.

Of these, since water may degrade long-term preservation property of the finally prepared germicidal antiseptic composition for dilution or precipitation preventing effect after dilution, depending on the hardness of water when in use, purified water or water for injection is preferably used.

In addition, the germicidal antiseptic composition for dilution of the present invention can contain, in addition to the aforementioned first to the fourth components, a water-soluble alcohol having 1 to 3 carbon atoms as a fifth component.

The fifth component in the germicidal antiseptic composition for dilution of the present invention only needs to be a pharmaceutically acceptable water-soluble alcohol having 1 to 3 carbon atoms. It is added to a concentration of not more than 10 w/v%, preferably not more than 5 w/v%, in the final germicidal antiseptic composition for dilution, whereby a precipitation preventing effect after dilution can be further improved without impairing the germicidal disinfection ability of the germicidal antiseptic composition for dilution of the present invention.

As the water-soluble alcohol having 1 to 3 carbon atoms, for example, alcohols such as methanol, ethanol, propanol and 2-propanol, and polyhydric alcohols such as propylene glycol, glycerol and the like can be mentioned. Of these, from the aspect of safety of the finally prepared germicidal antiseptic composition for dilution, ethanol, propanol, 2-propanol, propylene glycol (1,2-propanediol) and glycerol (1,2,3-propanetriol) are preferable. Of these, ethanol and 2-propanol are more preferable, and 2-propanol is particularly preferable.

The germicidal antiseptic composition for dilution of the present invention may contain, besides the aforementioned the first - the fifth components, germicidal ingredient other than chlorhexidine gluconate, stabilizers (macrogol 400, D-mannitol, D-SORBITOL etc.) and the like, coloring agents Red No. 2, Red No. 3, Red No. 102 and the like, tar colors designated by the Ministry of Health, Labour and Welfare regulation No. 127) used to indicate an external agent, flavor and the like, to the extent the precipitation preventing effect on chlorhexidine gluconate by the second component and third component is not directly affected.

As the stabilizer to be used for the germicidal antiseptic composition for dilution of the present invention, for example, macrogol and the like can be mentioned. Even among macrogols, some having a congeal point exceeding 35°C may reduce the action of the aforementioned second component and third component, or precipitate during preservation. When macrogol is to be contained, therefore, one having a congeal point of not more than 35°C is desirably used, like the aforementioned second component. The amount of its addition is preferably within the range of not more than 2 w/v%.

The germicidal antiseptic composition for dilution of the present invention can be prepared by a conventionally

employed preparation method of an aqueous preparation for pharmaceutical agent. While the method of addition of each component, stirring conditions and the like are not particularly limited, the following method is desirable to sufficiently exert a precipitation preventing effect on chlorhexidine gluconate, which is the purpose of the present invention.

(1) The second component is previously dissolved in water, which is the fourth component, within the range of 40-80°C.

(2) The first component (i.e., chlorhexidine gluconate), which is the main component, is added within the range of 40-60°C and the mixture is sufficiently stirred.

(3) The mixture is cooled to the range of 20-35°C and the third component and other components are added.

Moreover, the pH of the germicidal antiseptic composition for dilution of the present invention is generally adjusted to fall within the range of 4-7, in consideration of stability of chlorhexidine gluconate and safety to living organisms.

In this way, the precipitation of chlorhexidine gluconate upon dilution with hard water can be suppressed because the germicidal antiseptic composition for dilution of the present invention simultaneously contains a particular nonionic surfactant and a particular organic monocarboxylic acid. Consequently, the composition can be used widely depending on the purpose of use, as a germicidal antiseptic composition to be diluted when in use, which is free of limitation on the water to be used for dilution. As the water to be used for dilution, water having total hardness of not more than 300 mg/L, preferably not more than 200 mg/L, more preferably not more than 100 mg/L, is preferably used.

In the present description, the total hardness means a combination of permanent hardness and temporary hardness, which is a numerical value of the amount (mg/L) of divalent metal ion in water after conversion to the amount of calcium carbonate.

Furthermore, the germicidal antiseptic composition for dilution of the present invention can be used after dilution with water or ethanol to various concentrations depending on the purpose of use. The ethanol for dilution may be water-containing ethanol. To be specific, it can be used for, for example, disinfection of hands and skin, disinfection of the skin of operation site (operation region), disinfection of medical instruments, disinfection of wound site of skin, disinfection of operation room, patient's room etc., and the like. In this case, the composition is appropriately diluted and used to make the chlorhexidine gluconate concentration fall within the range of 0.05-0.5 w/v%.

## Examples

The germicidal antiseptic composition for dilution of the present invention is explained in more detail in the following by referring to Examples, which are not to be construed as limitative.

[Example 1]

According to the composition shown in Table 1, a germicidal antiseptic composition for dilution containing chlorhexidine gluconate (5 w/v%) was prepared.

An aqueous solution (20 mL) of polyoxyethylene(9)lauryl ether (Japanese Pharmacopoeia lauromacrogol) adjusted in advance with purified water to 10 w/v% was measured in a 200 mL vol glass beaker. Then, the mixture was heated to about 50°C, and chlorhexidine gluconate solution (the Japanese Pharmacopoeia) (25 mL) was added with stirring. The mixture was cooled to about 30°C, 25 w/v% aqueous solution (0.4 mL) of glucono-δ-lactbAe prepared in advance and 0.1 w/v% aqueous solution (5 mL) of Food Color Red No. 2 were added, and the mixture was stirred sufficiently. Using purified water, the total amount was finally adjusted to become 100 mL.

For the coloring agent, one widely used:in a chlorhexidine gluconate-containing germicidal antiseptic was used for indicating an external application. The pH after preparation was 4.8.

The chlorhexidine gluconate solution (the Japanese Pharmacopoeia) used for preparation contains 19.0-21.0 w/v% of chlorhexidine gluconate.

[Examples 2 - 22, Comparative Examples 1 - 38]

According to the compositions shown in Tables 1 - 12, a germicidal antiseptic liquid compositions for dilution were prepared.

The preparation method was the same as in Example 1.

In each composition, the surfactant was added in the amount shown in Table to give a 10 w/v% aqueous solution (20 w/v% aqueous solution for Example 12 alone). The organic monocarboxylic acid and macrogol were added in the amount shown in Table to give a 25 w/v% aqueous solution. The coloring agent (Food Color Red No. 2) was added in the amount shown in Table to give a 0.1 w/v% aqueous solution. Conc. glycerol, ethanol and 2-propanol were added in the amounts shown in Table without dilution. Then, these were stirred, mixed, and finally adjusted to 100 mL

with purified water to give preparations.

The results of the properties and pH measurement immediately after formulation of each preparation are respectively shown in Tables 1 - 12.

The HLB and congeal points of polyoxyethylene alkyl ether and other surfactants used for the formulation of each preparation are as shown in Table 13 and Table 14. In Tables, the number in the parentheses for each surfactant shows the number of moles of ethylene oxide addition or propylene oxide addition. Table 15 shows organic carboxylic acids and salts thereof used for the formulation.

In addition, glucono-δ-lactone used was of a food additive grade. Ethanol, 2-propanol, conc. glycerol, macrogols and disodium edetate used were the Japanese Pharmacopoeia compatible products. The coloring agent used was of a food additive grade. Other components were reagents manufactured by Nacalai Tesque. In all Examples and Comparative Examples, purified water was used for the formulation. The congeal point of macrogol 1540 used for the formulation was 46°C, and the congeal point of macrogol 4000 was 56°C.

Table 1

| component | Preparation | | | | |
|---|---|---|---|---|---|
| | Ex. 1 | Ex. 2 | Ex. 3 | Com. Ex. 1 | Com. Ex. 2 |
| chlorhexidine gluconate solution | 25 mL | 25 mL | 25 mL | 25 mL | 25 mL |
| polyoxyethylene(9) lauryl ether | 2 g | 2 g | 2 g | 2 g | 2 g |
| glucono-δ-lactone | 0.1 g | 0.15 g | - | - | - |
| acetic acid | - | - | 0.025 g | - | - |
| citric acid | - | - | - | - | 0.05g |
| Food Color Red No.2 | 5 mg | 5 mg | 5 mg | 5 mg | 5 mg |
| purified water | q.s. | q.s. | q.s. | q.s. | q.s. |
| total | 100 mL | 100 mL | 100 mL | 100 mL | 100 mL |
| properties immediately after formulation | clear red | clear red | clear red | clear red | clear red |
| pH immediately after formulation | 4.8 | 4.4 | 5.0 | 5.8 | 4.6 |

Table 2

| component | Preparation | | | | |
|---|---|---|---|---|---|
| | Ex. 4 | Ex. 5 | Ex. 6. | Ex. 7 | Com. Ex. 3 |
| chlorhexidine gluconate solution | 25 mL | 25 mL | 25 mL | 25 mL | 25 mL |
| polyoxyethylene(10)oleyl ether | 4 g | 4 g | 4 g | 2 g | 4 g |
| polyoxyethylene (9) lauryl ether | - | - | - | 2 g | - |
| glucono-δ-lactone | 0.1 g | 0.05 g | 0.1 g | - | - |
| acetic acid | - | - | - | 0.025 g | - |
| conc. glycerol | - | - | 1.0 g | - | - |
| 2-propanol | - | 4 mL | - | 4 mL | 4 mL |
| Food Color Red No.2 | 5 mg | 5 mg | 5 mg | 5 mg | 5 mg |
| purified water | q.s. | q.s. | q.s. | q.s. | q.s. |
| total | 100 mL | 100 mL | 100 mL | 100 mL | 100 mL |
| properties immediately after formulation | clear red | clear red | clear red | clear red | clear red |
| pH immediately after formulation | 4.8 | 4.9 | 4.8 | 4.9 | 5.8 |

Table 3

| component | Preparation | | | | |
|---|---|---|---|---|---|
| | Ex. 8 | Ex. 9 | Ex. 10 | Ex. 11 | Ex. 12 |
| chlorhexidine gluconate solution | 25 mL | 25 mL | 25 mL | 25 mL | 25 mL |
| polyoxyethylene(10) oleyl ether | 5 g | 5 g | - | 2.5 g | 7.5 g |
| polyoxyethylene(9) lauryl ether | - | - | 3 g | 2.5 g | - |
| glucono-δ-lactone | 0.05 g | 0.075 g | 0.1 g | - | 0.1 g |
| acetic acid | - | - | - | 0.025 g | - |
| D-mannitol | - | - | 0.2 g | - | - |
| 2-propanol | 4 mL | 4 mL | 4 mL | - | - |
| Food Color Red No.2 | 5 mg | 5 mg | 5 mg | 5 mg | 5 mg |
| purified water | q.s. | q.s. | q.s. | q.s. | q.s. |
| total | 100 mL | 100 mL | 100 mL | 100 mL | 100 mL |
| properties immediately after formulation | clear red | clear red | clear red | clear red | clear red |
| pH immediately after formulation | 4.9 | 4.8 | 4.8 | 4.9 | 4.8 |

Table 4

| component | Preparation | | | | |
|---|---|---|---|---|---|
| | Ex. 13 | Ex. 14 | Com. Ex. 4 | Com. Ex. 5 | Com. Ex. 6 |
| chlorhexidine gluconate solution | 25 mL | 25 mL | 25 mL | 25 mL | 25 mL |
| polyoxyethylene(10)oleyl ether | 4 g | 4 g | 4 g | 4 g | 4 g |
| glucono-δ-lactone | 0.02 g | 0.05 g | - | - | - |
| acetic acid | 0.01 g | - | - | - | - |
| citric acid | - | - | 0.1 g | | 0.1 g |
| tartaric acid | - | - | - | 0.05 g | |
| trisodium citrate | - | - | - | | 0.4 g |
| Macrogol 400 | - | 1.0 g | - | - | - |
| 2-propanol | 2 mL | 2 mL | 2 mL | 2 mL | 2 mL |
| Food Color Red No.2 | 5 mg | 5 mg | 5 mg | 5 mg | 5 mg |
| purified water | q.s. | q.s. | q.s. | q.s. | q.s. |
| total | 100 mL | 100 mL | 100 mL | 100 mL | 100 mL |
| properties immediately after formulation | clear red | clear red | clear red | clear red | clouded |
| pH immediately after formulation | 5.0 | 4.9 | 4.4 | 4.5 | |

Table 5

| component | Preparation | | | | |
|---|---|---|---|---|---|
| | Com. Ex. 7 | Com. Ex. 8 | Com. Ex. 9 | Com. Ex. 10 | Com. Ex. 11 |
| chlorhexidine gluconate solution | 25 mL | 25 mL | 25 mL | 25 mL | 25 mL |
| polyoxyethylene (9) lauryl ether | 2 g | 2 g | 2 g | 2 g | 2 g |
| citric acid | 0.1 g | - | - | - | - |
| disodium tartrate | 0.5 g | 0.5 g | | - | - |
| disodium malate | - | - | 0.2 g | | |
| sodium gluconate | - | - | - | 0.5 g 0.5 g | - |
| sodium acetate | - | - | - | - | 0.1 g |
| 2-propanol | 4 mL | 4 mL | 4 mL | 4 mL | 4 mL |
| Food Color Red No.2 | 5 mg | 5 mg | 5 mg | 5 mg | 5 mg |
| purified water | q.s. | q.s. | q.s. | q.s. | q.s. |
| total | 100 mL | 100 mL | 100 mL | 100 mL | 100 mL |
| properties immediately after formulation | clear red | clear red | clear red | clear red | clear red |
| pH immediately after formulation | 4.6 | 6.0 | 5.9 | 5.9 | 6.1 |

Table 6

| component | Preparation | | | | |
|---|---|---|---|---|---|
| | Ex. 15 | Ex. 16 | Ex. 17 | Com. Ex. 12 | Com. Ex. 13 |
| chlorhexidine gluconate solution | 25 mL | 25 mL | 25 mL | 25 mL | 25 mL |
| polyoxyethylene(7)lauryl ether | 3 g g | - | - | - | - |
| polyoxyethylene (11) lauryl ether | - | 3 g | 5 g | - | - |
| polyoxyethylene(20)lauryl ether | - | - | - | 3 g g | - |
| polyoxyethylene(30)lauryl ether | - | - | - | - | 3 g |
| glucono-δ-lactone | 0.1 g | 0.1 g | 0.1 g | 0.1 g | 0.1 g |
| calcium gluconate | - | - | 0.2 g | - | - |
| ethanol | 4 mL | 4 mL | 4 mL | 4 mL | 4 mL |
| Food Color Red No.2 | 5 mg | 5 mg | 5 mg | 5 mg | 5 mg |
| purified water | q.s. | q.s. | q.s. | q.s. | q.s. |
| total | 100 mL | 100 mL | 100 mL | 100 mL | 100 mL |
| properties immediately after formulation | clear red | clear red | clear red | clear red | clear red |
| pH immediately after formulation | 4.8 | 4.8 | 4.8 | 4.8 | 4.8 |

Table 7

| component | Preparation | | | | |
|---|---|---|---|---|---|
| | Ex. 18 | Ex. 19 | Ex. 20 | Ex. 21 | Ex. 22 |
| chlorhexidine gluconate solution | 25 mL | 25 mL | 25 L | 25 mL | 25 mL |

Table continued

| component | Preparation | | | | |
|---|---|---|---|---|---|
| | Ex. 18 | Ex. 19 | Ex. 20 | Ex. 21 | Ex. 22 |
| polyoxyethylene (7) oleyl ether | 4 g | - | - | 2 g | 2 g |
| polyoxyethylene (12) oleyl ether | - | 4 g | - | 2g | - |
| polyoxyethylene(15)oleyl ether | - | - | 4 g | - | - |
| polyoxyethylene(20)oleyl ether | - | - | - | - | 2 g |
| glucono-δ-lactone | 0.1 g | 0.05 g | 0.1 g | 0.1 g | - |
| acetic acid | - | - | - | - | 0.025 g |
| Food Color Red No.2 | 5 mg | 5 mg | 5 mg | 5 mg | 5 mg |
| purified water | q.s. | q.s. | q.s. | q.s. | q.s. |
| total | 100 mL | 100 mL | 100 mL | 100 mL | 100 mL |
| properties immediately after formulation | clear red | clear red | clear red | clear red | clear red |
| pH immediately after formulation | 4.8 | 4.9 | 4.8 | 4.8 | 4.9 |

Table 8

| component | Preparation | | | | |
|---|---|---|---|---|---|
| | Com. Ex. 14 | Com. Ex. 15 | Com. Ex. 16 | Com. Ex. 17 | Com. Ex. 18 |
| chlorhexidine gluconate solution | 25 mL | 25 mL | 25 mL | 25 mL | 25 mL |
| polyoxyethylene(13)cetyl ether | 4 g | 4 g | 4 g | - | - |
| polyoxyethylene (20)-polyoxypropylene (8)-cetyl ether | | - | - | 4 g | 4 g |
| glucono-δ-lactone | - | 0.1 g | - | - | 0.1 g |
| acetic acid | - | - | 0.025 g | - | - |
| 2-propanol | 4 mL | 4 mL | 4 mL | 4 mL | 4 mL |
| Food Color Red No.2 | 5 mg | 5 mg | 5 mg | 5 mg | 5 mg |
| purified water | q.s. | q.s. | q.s. | q.s. | q.s. |
| total | 100 mL | 100 mL | 100 mL | 100 mL | 100 mL |
| properties immediately after formulation | clear red | clear red | clear red | clear red | clear red |
| pH immediately after formulation | 5 8 | 4 8 | 5.0 | 5.9 | 4.8 |

Table 9

| component | Preparation | | | | |
|---|---|---|---|---|---|
| | Com. Ex. 19 | Com. Ex. 20 | Com. Ex. 21 | Com. Ex. 22 | Com. Ex. 23 |
| chlorhexidine gluconate solution | 25 mL | 25 mL | 25 mL | 25 mL | 25 mL |
| polyoxyethylene(20)-polyoxypropylene(20)-glycol | 4 g | 4 g | - | - | - |
| polyoxyethylene(196)-polyoxypropylene(67)-glycol | - | - | 4 g | 4 g | - |
| polyoxyethylene(160)-polyoxypropylene(30)-glycol | - | - | - | - | 4 g |

Table continued

| component | Preparation | | | | |
|---|---|---|---|---|---|
| | Com. Ex. 19 | Com. Ex. 20 | Com. Ex. 21 | Com. Ex. 22 | Com. Ex. 23 |
| glucono-δ-lactone | - | 0.1 g | - | 0.1 g | 0.1 g |
| 2-propanol | 4 mL | 4 mL | 4 mL | 4 mL | 4 mL |
| Food Color Red No.2 | 5 mg | 5 mg | 5 mg | 5 mg | 5 mg |
| purified water | q.s. | q.s. | q.s. | q.s. | q.s. |
| total | 100 mL | 100 mL | 100 mL | 100 mL | 100 mL |
| properties immediately after formulation | clear red | clear red | clear red | clear red | clear red |
| pH immediately after. formulation | 5.8 | 4.8 | 5.9 | 4.8 | 4.9 |

Table 10

| component | Preparation | | | | |
|---|---|---|---|---|---|
| | Com. Ex. 24 | Com. Ex. 25 | Com. Ex. 26 | Com. Ex. 27 | Com. Ex. 28 |
| chlorhexidine gluconate solution | 25 mL | 25 mL | 25 mL | 25 mL | 25 mL |
| Macrogol 400 | 4 g | - | - | - | - |
| Macrogol 1540 | - | 4 g | - | - | - |
| Macrogol 4000 | - | - | 2 g | - | - |
| glucono-δ-lactone | 0.1 g | 0.1 g | 0.1 g | 0.1 g | - |
| acetic acid | - | - | - | - | 0.025 g |
| 2-propanol | 4 mL | 4 mL | 4 mL | 4 mL | 4 mL |
| Food Color Red No.2 | 5 mg | mg | 5 mg | 5 mg | 5 mg |
| purified water | q.s. | q.s. | q.s. | q.s. | q.s. |
| total | 100 mL | 100 mL | 100 mL | 100 mL | 100 mL |
| properties immediately after red formulation | clear red | clear | clear red | clear red | clear red |
| pH immediately after formulation | 4.8 | 4.9 | 4.8 | 4.8 | 4.8 |

Table 11

| component | Preparation | | | | |
|---|---|---|---|---|---|
| | Com. Ex. 29 | Com. Ex. 30 | Com. Ex. 31 | Com. Ex. 32 | Com. Ex. 33 |
| chlorhexidine gluconate solution | 25 mL | 25 mL | 25 mL | 25 mL | 25 mL |
| glucono-δ-lactone | - | - | 0.1 g | - | 0.1 g |
| tartaric acid | 0.1 g | - | - | - | - |
| disodium tartrate | - | 0.2 g | - | - | - |
| disodium malate | - | - | 0.2 g | - | - |
| citric acid | - | - | - | 0.1 g | - |
| trisodium citrate | - | - | - | - | 0.5 g |
| 2-propanol | 4 mL | 4 mL | 4 mL | 4 mL | 4 mL |
| Food Color Red No.2 | 5 mg | 5 mg | 5 mg | 5 mg | 5 mg |
| purified water | q.s. | q.s. | q.s. | q.s. | q.s. |
| total | 100 mL | 100 mL | 100 mL | 100 mL | 100 mL |
| properties immediately after formulation | clear red | clouded | clouded | clouded | clouded |
| pH immediately after formulation | 4.3 | | | | |

Table 12

| component | Preparation | | | | |
|---|---|---|---|---|---|
| | Com. Ex. 34 | Com. Ex. 35 | Com. Ex. 36 | Com. Ex. 37 | Com. Ex.38 |
| chlorhexidine gluconate solution | 25 mL | 25 mL | 25 mL | 25 mL | 25 mL |
| polyoxyethylene(9) lauryl ether | - | 3 g | - | 3 g | 3 g |
| sodium lauryl sulfate | 2 g | 2 g | - | - | - |
| sodium stearate | - | - | 2 g | 2 g | - |
| glucono-δ-lactone | 0.1 g | 0.1 g | 0.1 g | 0.1 g | - |
| disodium edetate | - | - | - | - | 0.1 g |
| 2-propanol | 4 mL | 4 mL | 4 mL | 4 mL | 4 mL |
| Food Color Red No.2 | 5 mg | 5 mg | 5 mg | 5 mg | 5 mg |
| purified water | q.s. | q.s. | q.s. | q.s. | q.s. |
| total | 100 mL | 100 mL | 100 mL | 100 mL | 100 mL |
| properties immediately after formulation | clouded | clouded | clouded | clouded | clouded |
| pH immediately after formulation | / | / | / | / | / |

Table 13 Surfactant (1) used for formulation

| surfactant | grade | congeal point (°C) | HLB* |
|---|---|---|---|
| polyoxyethylene(7)lauryl ether | Japanese Pharmacopoeia | 10 | 12.5 |
| polyoxyethylene(9)lauryl ether | Japanese Pharmacopoeia | 22 | 13.6 |
| polyoxyethylene (11) lauryl ether | Japanese Pharmacopoeia | 26 | 14.5 |
| polyoxyethylene(20)lauryl ether | Japanese Pharmacopoeia | 34 | 16.5 |
| polyoxyethylene(30)lauryl ether | Japanese Pharmacopoeia | 43 | 17.5 |
| polyoxyethylene(7)oleyl ether | Japanese Pharmaceutical Excipient | 18 | 10.7 |
| polyoxyethylene(10)oleyl ether | Japanese Pharmaceutical Excipient | 23 | 12.5 |
| polyoxyethylene(12)oleyl | Japanese Pharmaceutical Excipient | 25 | 13.3 |
| polyoxyethylene(15)oleyl ether | Japanese Pharmaceutical Excipient | 28 | 14.2 |
| polyoxyethylene(20)oleyl ether | Japanese Pharmaceutical Excipient | 35 | 15.4 |
| *) Tokiyuki Yoshida et al., new Surfactant Handbook, Kougakutosho (1987) | | | |

Table 14 Surfactant (2) used for formulation

| surfactant | grade | congeal point (°C) | HLB* |
|---|---|---|---|
| polyoxyethylene(13)cetyl ether | Japanese Pharmaceutical Excipient | 36 | 14.1 |
| polyoxyethylene(20)-polyoxypropylene(8)-cetyl ether | Japanese Pharmaceutical Excipient | 37 | 14.8 |

Table continued

| surfactant | grade | congeal point (°C) | HLB* |
|---|---|---|---|
| polyoxyethylene(20)-polyoxypropylene(20)-glycol | Japanese Pharmaceutical Excipient | 20 not more than | 8.6 |
| polyoxyethylene(160)-polyoxypropylene(30)-glycol | Japanese Pharmaceutical Excipient | 50 | 13.8 |
| polyoxyethylene(196)-polyoxypropylene(67)-glycol | Japanese Pharmaceutical Excipient | 56 | 16.0 |
| sodium lauryl sulfate | Japanese Pharmaceutical Excipient | - | about 40 |
| sodium stearate | Japanese Pharmaceutical Excipient | - | 18 |
| *) Tokiyuki Yoshida et al., new Surfactant Handbook, Kougakutosho (1987) | | | |

Table 15 Molecular weight of organic carboxylic acid or a salt thereof used for formulation

| organic carboxylic acid or a salt thereof | | molecular weight |
|---|---|---|
| composition name | reagent used | |
| glucono-δ-lactone | glucono-δ-lactone | 178.14 |
| acetic acid | acetic acid | 60.05 |
| sodium gluconate | Sodium gluconate | 218.14 |
| calcium gluconate | calcium gluconate monohydrate | 448.39 |
| sodium acetate | sodium acetate trihydrate | 136.08 |
| citric acid | citric acid monohydrate | 210.14 |
| trisodium citrate | Trisodium citrate dihydrate | 294.10 |
| tartaric acid | L-tartaric acid | 150.09 |
| disodium tartrate | disodium tartrate dihydrate | 230.08 |
| disodium malate | disodium DL-malate 1/2 hydrate | 187.06 |

As a result of formulation, clear solution were obtained in Examples 1 -22 and Comparative Examples 1 -5, Comparative Examples 7 -29, but the solution became clouded during formulation in Comparative Example 6 and Comparative Examples 30-38, and ultimately, clear solution could not be obtained.

From the foregoing results, it is clear that water-soluble polycarboxylic acid or a salt thereof, an organic chelating agent, an anionic surfactant and the like are difficult to add for the formulation of a germicidal antiseptic composition for dilution containing chlorhexidine gluconate, which is the object of the present invention.

[Experimental Example 1] Preservation test

Of the formulated respective germicidal antiseptic compositions for dilution, with regard to Examples 1 - 22, Comparative Examples 1 - 5 and Comparative Examples 7 - 29 wherein clear solutions were obtained, the formulated preparations were placed in transparent glass sample tubes and preserved in a cold preserving container at 5°C for 7 days, after which the properties were examined. The results are shown in Tables 16 and 17.

Table 16 Example

| Preparation | appearance | | Preparation | appearance | |
|---|---|---|---|---|---|
| | immediately after formulation | after preservation at 5°C for 7 days | | immediately after formulation | after preservation at 5°C for 7 days |
| 1 | clear red | clear red | 2 | clear red | clear red |
| 3 | clear red | clear red | 4 | clear red | clear red |
| 5 | clear red | clear red | 6 | clear red | clear red |
| 7 | clear red | clear red | 8 | clear red | clear red |
| 9 | clear red | clear red | 10 | clear red | clear red |
| 11 | clear red | clear red | 12 | clear red | clear red |
| 13 | clear red | clear red | 14 | clear red | clear red |
| 15 | clear red | clear red | 16 | clear red | clear red |
| 17 | clear red | clear red | 18 | clear red | clear red |
| 19 | clear red | clear red | 20 | clear red | clear red |
| 21 | clear red | clear red | 22 | clear red | clear red |

Table 17 Comparative Example

| Preparation | appearance | | Preparation | appearance | |
|---|---|---|---|---|---|
| | immediately after formulation | after preservation at 5°C for 7 days | | Immediately after formulation | after preservation at 5°C for 7 days |
| 1 | clear red | clear red | 2 | clear red | clear red |
| 3 | clear red | clear red | 4 | clear red | clear red |
| 5 | clear red | clear red | 7 | clear red | solid precipitate |
| 8 | clear red | clear red | 9 | clear red | solid precipitate |
| 10 | clear red | clear red | 11 | clear red | clear red |
| 12 | clear red | clear red | 13 | clear red | clear clear |
| 14 | clear red | clear red | 15 | clear red | clear red |
| 16 | clear red | clear red | 17 | clear red | clear red |
| 18 | clear red | clear red | 19 | clear red | clear red |
| 20 | clear red | clear red | 21 | clear red | clear red |
| 22 | clear red | clear red | 23 | clear red | clear red |
| 24 | clear red | clear red | 25 | clear red | solid precipitate |
| 26 | clear red | solid precipitate | 27 | clear red | clear red |
| 28 | clear red | red | 29 | clear red | crystal precipitate |

As a result, in Comparative Examples 7 and 9 containing polycarboxylic acid and polycarboxylic acid salts, and Comparative Examples 25 and 26 containing polyethylene glycol having a high congeal point, precipitation of solids was observed. In Comparative Example 29 where a surfactant was not added but tartaric acid, which is a'polycarboxylic acid, was added, crystal precipitation was observed.

From the foregoing results, it is clear that polycarboxylic acid (salt) and polyalkylene glycol having a high congeal point are difficult to add for the formulation of a germicidal antiseptic composition for dilution containing chlorhexidine gluconate, which is the object of the present invention.

[Experimental Example 2] Dilution test

Of the prepared respective germicidal antiseptic compositions for dilution, Examples 1 - 22, and Comparative Examples 1 - 5 and 7 - 29 obtained as clear solutions during preparation were subjected to a dilution test using two kinds of model water.

To be specific, model water was placed in a transparent glass sample tube, and each germicidal antiseptic composition for dilution was added to 50-fold dilution. The mixture was immediately stirred with a test tube mixer for 10 seconds, and precipitation during standing still was visually observed with. time at room temperature. The test was performed with regard to 5 test tubes for each preparation. The precipitation score is the number of sample tubes out of 5, which showed precipitation.

As model water 1, water adjusted to have the total hardness of about 285 mg/L, which is near the standard upper limit for tap water in Japan (not more than 300 mg/L), was used. The results thereof are shown in Table 18 - Table 21.

The model water 1 used contained the following ions.

calcium ion: 74.5 mg/L
magnesium ion: 24.8 mg/L
sodium ion: 78.7 mg/L
chlorine ion: 158.6 mg/L
sulfate ion: 218.5 mg/L
nitrate ion: 9.7 mg/L

Using model water 2 obtained by diluting the aforementioned model water 1 3-fold with purified water to adjust the total hardness to about 95 mg/L, a dilution test was performed in the same manner, and precipitation was observed. The results are shown in Table 22 - Table 25.

As is evident from the results of Table 18 - Table 25, of the germicidal antiseptic compositions for dilution prepared as clear solutions, the use of the second component surfactant alone, the use of the third component organic monocarboxylic acid alone, and a combined use of the second component surfactant, and carboxylic acid or a salt thereof other than the third component failed to sufficiently prevent the precipitation. In contrast, germicidal antiseptic composition for dilution of the present invention showed a markedly improved precipitation suppressing effect when the second component surfactant and the third component organic monocarboxylic acid were simultaneously used.

The foregoing results establish that the germicidal antiseptic composition for dilution of the present invention has high stability against precipitation of chlorhexidine gluconate when diluted with water having a hardness of tap water.

Table 18 Precipitation score (1) of germicidal antiseptic compositions for dilution (model water 1 was used, 50-fold dilution)

| Preparation | | Precipitation score for each standing time after dilution | | | | | | | | |
|---|---|---|---|---|---|---|---|---|---|---|
| | | within 30 min | 1 hr later | 2 hrs later | 3 hrs later | 4 hrs later | 5 hrs later | 6 hrs later | 8 hrs later | 24 hrs later |
| No. Ex. | 1 | 0 | 0 | 0 | 0 | 0 | 0 | 0 | 0 | 0 |
| | 2 | 0 | 0 | 0 | 0 | 0 | 0 | 0 | 0 | 0 |
| | 3 | 0 | 0 | 0 | 0 | 0 | 0 | 0 | 1 | 1 |
| | 4 | 0 | 0 | 0 | 0 | 0 | 0 | 0 | 0 | 1 |
| | 5 | 0 | 0 | 0 | 0 | 0 | 0 | 0 | 0 | 0 |
| | 6 | 0 | 0 | 0 | 0 | 0 | 0 | 0 | 0 | 0 |
| | 7 | 0 | 0 | 0 | 0 | 0 | 0 | 0 | 0 | 1 |
| | 8 | 0 | 0 | 0 | 0 | 0 | 0 | 0 | 0 | 0 |
| | 9 | 0 | 0 | 0 | 0 | 0 | 0 | 0 | 0 | 0 |
| | 10 | 0 | 0 | 0 | 0 | 0 | 0 | 0 | 0 | 0 |

Table 19 Precipitation score (2) of germicidal antiseptic compositions for dilution (model water 1 was used, 50-fold dilution)

| Preparation | | Precipitation score for each standing time after dilution | | | | | | | | |
|---|---|---|---|---|---|---|---|---|---|---|
| | | Within 30 min | 1 hr later | 2 hrs later | 3 hrs later | 4 hrs later | 5 hrs later | 6 hrs later | 8 hrs later | 24 hrs later |
| No. Ex. | 11 | 0 | 0 | 0 | 0 | 0 | 0 | 0 | 0 | 0 |
| | 12 | 0 | 0 | 0 | 0 | 0 | 0 | 0 | 0 | 0 |
| | 13 | 0 | 0 | 0 | 0 | 0 | 0 | 0 | 0 | 1 |
| | 14 | 0 | 0 | 0 | 0 | 0 | 0 | 0 | 0 | 0 |
| | 15 | 0 | 0 | 0 | 0 | 0 | 0 | 0 | 0 | 0 |
| | 16 | 0 | 0 | 0 | 0 | 0 | 0 | 0 | 0 | 1 |
| | 17 | 0 | 0 | 0 | 0 | 0 | 0 | 0 | 0 | 1 |
| | 18 | 0 | 0 | 0 | 0 | 0 | 0 | 0 | 0 | 1 |
| | 19 | 0 | 0 | 0 | 0 | 0 | 0 | 0 | 0 | 1 |
| | 20 | 0 | 0 | 0 | 0 | 0 | 0 | 0 | 0 | 1 |
| | 21 | 0 | 0 | 0 | 0 | 0 | 0 | 0 | 0 | 1 |
| | 22 | 0 | 0 | 0 | 0 | 0 | 0 | 0 | 1 | 1 |

Table 20 Precipitation score (3) of germicidal antiseptic compositions for dilution (model water 1 was used, 50-fold dilution)

| Preparation No. | | Precipitation score for each standing time after dilution | | | | | | | | |
|---|---|---|---|---|---|---|---|---|---|---|
| | | within 30 min | 1 hr later | 2 hrs later | 3 hrs later | 4 hrs later | 5 hrs later | 6 hrs later | 8 hrs later | 24 hrs later |
| Com. Ex. | 1 | 0 | 0 | 0 | 0 | 0 | 1 | 2 | 4 | 5 |
| | 2 | 0 | 0 | 5 | 5 | 5 | 5 | 5 | 5 | 5 |
| | 3 | 0 | 0 | 0 | 0 | 0 | 0 | 2 | 5 | 5 |
| | 4 | 0 | 0 | 5 | 5 | 5 | 5 | 5 | 5 | 5 |
| | 5 | 0 | 0 | 1 | 2 | 2 | 3 | 4 | 5 | 5 |
| | 7 | 0 | 1 | 3 | 5 | 5 | 5 | 5 | 5 | 5 |
| | 8 | 0 | 0 | 1 | 2 | 4 | 5 | 5 | 5 | 5 |
| | 9 | 0 | 0 | 1 | 2 | 5 | 5 | 5 | 5 | 5 |
| | 10 | 0 | 0 | 0 | 0 | 2 | 3 | 4 | 5 | 5 |
| | 11 | 0 | 0 | 0 | 0 | 0 | 1 | 3 | 5 | 5 |
| | 12 | 0 | 0 | 2 | 4 | 5 | 5 | 5 | 5 | 5 |
| | 13 | 0 | 5 | 5 | 5 | 5 | 5 | 5 | 5 | 5 |
| | 14 | 5 | 5 | 5 | 5 | 5 | 5 | 5 | 5 | 5 |
| | 15 | 5 | 5 | 5 | 5 | 5 | 5 | 5 | 5 | 5 |

Table 21 Precipitation score (4) of germicidal antiseptic compositions for dilution (model water 1 was used, 50-fold dilution)

| Preparation No. | | Precipitation score for each standing time after dilution | | | | | | | | |
|---|---|---|---|---|---|---|---|---|---|---|
| | | within 30 min | 1 hr later | 2 hrs later | 3 hrs later | 4 hrs later | 5 hrs later | 6 hrs later | 8 hrs later | 24 hrs later |
| Com. Ex. | 16 | 5 | 5 | 5 | 5 | 5 | 5 | 5 | 5 | 5 |
| | 17 | 0 | 0 | 5 | 5 | 5 | 5 | 5 | 5 | 5 |
| | 18 | 0 | 0 | 5 | 5 | 5 | 5 | 5 | 5 | 5 |
| | 19 | 5 | 5 | 5 | 5 | 5 | 5 | 5 | 5 | 5 |
| | 20 | 5 | 5 | 5 | 5 | 5 | 5 | 5 | 5 | 5 |
| | 21 | 2 | 5 | 5 | 5 | 5 | 5 | 5 | 5 | 5 |
| | 22 | 2 | 5 | 5 | 5 | 5 | 5 | 5 | 5 | 5 |
| | 23 | 3 | 5 | 5 | 5 | 5 | 5 | 5 | 5 | 5 |
| | 24 | 5 | 5 | 5 | 5 | 5 | 5 | 5 | 5 | 5 |
| | 25 | 5 | 5 | 5 | 5 | 5 | 5 | 5 | 5 | 5 |
| | 26 | 5 | 5 | 5 | 5 | 5 | 5 | 5 | 5 | 5 |
| | 27 | 5 | 5 | 5 | 5 | 5 | 5 | 5 | 5 | 5 |
| | 28 | 5 | 5 | 5 | 5 | 5 | 5 | 5 | 5 | 5 |
| | 29 | 5 | 5 | 5 | 5 | 5 | 5 | 5 | 5 | 5 |

Table 22 Precipitation score (5) of germicidal antiseptic compositions for dilution (model water 2 was used, 50-fold dilution)

| Preparation No. | | Precipitation score for each standing time after dilution | | | | | | | | |
|---|---|---|---|---|---|---|---|---|---|---|
| | | within 30 min | 1 hr later | 2 hrs later | 3 hrs later | 4 hrs later | 5 hrs later | 6 hrs later | 8 hrs later | 24 hrs later |
| Ex. | 1 | 0 | 0 | 0 | 0 | 0 | 0 | 0 | 0 | 0 |
| | 2 | 0 | 0 | 0 | 0 | 0 | 0 | 0 | 0 | 0 |
| | 3 | 0 | 0 | 0 | 0 | 0 | 0 | 0 | 0 | 0 |
| | 4 | 0 | 0 | 0 | 0 | 0 | 0 | 0 | 0 | 0 |
| | 5 | 0 | 0 | 0 | 0 | 0 | 0 | 0 | 0 | 0 |
| | 6 | 0 | 0 | 0 | 0 | 0 | 0 | 0 | 0 | 0 |
| | 7 | 0 | 0 | 0 | 0 | 0 | 0 | 0 | 0 | 0 |
| | 8 | 0 | 0 | 0 | 0 | 0 | 0 | 0 | 0 | 0 |
| | 9 | 0 | 0 | 0 | 0 | 0 | 0 | 0 | 0 | 0 |
| | 10 | 0 | 0 | 0 | 0 | 0 | 0 | 0 | 0 | 0 |
| | 11 | 0 | 0 | 0 | 0 | 0 | 0 | 0 | 0 | 0 |

Table 23 Precipitation score (6) of germicidal antiseptic compositions for dilution (model water 2 was used, 50-fold dilution)

| Preparation No. | | Precipitation score for each standing time after dilution | | | | | | | | |
|---|---|---|---|---|---|---|---|---|---|---|
| | | within 30 min | 1 hr later | 2 hrs later | 3 hrs later | 4 hrs later | 5 hrs later | 6 hrs later | 8 hrs later | 24 hrs later |
| Ex. | 12 | 0 | 0 | 0 | 0 | 0 | 0 | 0 | 0 | 0 |
| | 13 | 0 | 0 | 0 | 0 | 0 | 0 | 0 | 0 | 0 |
| | 14 | 0 | 0 | 0 | 0 | 0 | 0 | 0 | 0 | 0 |
| | 15 | 0 | 0 | 0 | 0 | 0 | 0 | 0 | 0 | 0 |
| | 16 | 0 | 0 | 0 | 0 | 0 | 0 | 0 | 0 | 0 |
| | 17 | 0 | 0 | 0 | 0 | 0 | 0 | 0 | 0 | 0 |
| | 18 | 0 | 0 | 0 | 0 | 0 | 0 | 0 | 0 | 0 |
| | 19 | 0 | 0 | 0 | 0 | 0 | 0 | 0 | 0 | 0 |
| | 20 | 0 | 0 | 0 | 0 | 0 | 0 | 0 | 0 | 0 |
| | 21 | 0 | 0 | 0 | 0 | 0 | 0 | 0 | 0 | 0 |
| | 22 | 0 | 0 | 0 | 0 | 0 | 0 | 0 | 0 | 1 |

Table 24 Precipitation score (7) of germicidal antiseptic compositions for dilution (model water 2 was used, 50-fold dilution)

| Preparation No. | | Precipitation score for each standing time after dilution | | | | | | | | |
|---|---|---|---|---|---|---|---|---|---|---|
| | | within 30 min | 1 hr later | 2 hrs later | 3 hrs later | 4 hrs later | 5 hrs later | 6 hrs later | 8 hrs later | 24 hrs later |
| Com. Ex. | 1 | 0 | 0 | 0 | 0 | 0 | 1 | 2 | 3 | 4 |
| | 2 | 0 | 0 | 1 | 1 | 2 | 3 | 4 | 5 | 5 |
| | 3 | 0 | 0 | 0 | 0 | 0 | 0 | 1 | 3 | 4 |
| | 4 | 0 | 0 | 0 | 0 | 1 | 2 | 4 | 5 | 5 |
| | 5 | 0 | 0 | 1 | 2 | 2 | 3 | 4 | 5 | 5 |
| | 7 | 0 | 0 | 1 | 2 | 5 | 5 | 5 | 5 | 5 |
| | 8 | 0 | 0 | 0 | 1 | 2 | 3 | 4 | 5 | 5 |
| | 9 | 0 | 0 | 0 | 0 | 2 | 2 | 3 | 5 | 5 |
| | 10 | 0 | 0 | 0 | 0 | 1 | 1 | 4 | 4 | 4 |
| | 11 | 0 | 0 | 0 | 0 | 0 | 1 | 2 | 3 | 4 |
| | 12 | 0 | 0 | 1 | 2 | 3 | 4 | 5 | 5 | 5 |
| | 13 | 0 | 1 | 2 | 3 | 5 | 5 | 5 | 5 | 5 |
| | 14 | 0 | 5 | 5 | 5 | 5 | 5 | 5 | 5 | 5 |
| | 15 | 0 | 5 | 5 | 5 | 5 | 5 | 5 | 5 | 5 |

Table 25 Precipitation score (8) of germicidal antiseptic compositions for dilution (model water 2 was used, 50-fold dilution)

| Preparation No. | | Precipitation score for each standing time after dilution | | | | | | | | |
|---|---|---|---|---|---|---|---|---|---|---|
| | | within 30 min | 1 hr later | 2 hrs later | 3 hrs later | 4 hrs later | 5 hrs later | 6 hrs later | 8 hrs later | 24 hrs later |
| Com. Ex. | 16 | 0 | 5 | 5 | 5 | 5 | 5 | 5 | 5 | 5 |
| | 17 | 0 | 0 | 5 | 5 | 5 | 5 | 5 | 5 | 5 |
| | 18 | 0 | 0 | 5 | 5 | 5 | 5 | 5 | 5 | 5 |
| | 19 | 0 | 5 | 5 | 5 | 5 | 5 | 5 | 5 | 5 |
| | 20 | 0 | 5 | 5 | 5 | 5 | 5 | 5 | 5 | 5 |
| | 21 | 0 | 2 | 5 | 5 | 5 | 5 | 5 | 5 | 5 |
| | 22 | 0 | 2 | 5 | 5 | 5 | 5 | 5 | 5 | 5 |
| | 23 | 0 | 3 | 5 | 5 | 5 | 5 | 5 | 5 | 5 |
| | 24 | 5 | 5 | 5 | 5 | 5 | 5 | 5 | 5 | 5 |
| | 25 | 5 | 5 | 5 | 5 | 5 | 5 | 5 | 5 | 5 |
| | 26 | 5 | 5 | 5 | 5 | 5 | 5 | 5 | 5 | 5 |
| | 27 | 5 | 5 | 5 | 5 | 5 | 5 | 5 | 5 | 5 |
| | 28 | 5 | 5 | 5 | 5 | 5 | 5 | 5 | 5 | 5 |
| | 29 | 5 | 5 | 5 | 5 | 5 | 5 | 5 | 5 | 5 |

[Experimental Example 3] pH stability test

Each preparation of Example 1, Example 5, Example 7, Comparative Example 1 and Comparative Example 3 was placed in a shielding polyethylene bottle and the bottle was tightly sealed, which was followed by pH stability tests under preservation at 40°C and 50°C. The results are shown in Tables 26 and 27.

As is clear from Tables 26 and 27, Example 1, Example 5 and Example 7 hardly showed variation in pH value during the test period and were stable. In contrast, Comparative Example 1 and Comparative Example 3 showed greater variation in pH value as compared to Examples during the test period under any conditions. From these facts, it is clear that the germicidal antiseptic composition for dilution of the present invention is stable as a preparation.

Table 26 pH change of each preparation under 40°C preservation

| preparation formulation | initial value | 40°C preservation | | |
|---|---|---|---|---|
| | | 1 month later | 3 months later | 6 months later |
| Ex. 1 | 4.8 | 4.9 | 4.9 | 5.1 |
| Ex. 5 | 4.9 | 4.9 | 4.9 | 5.0 |
| Ex. 7 | 4.9 | 5.0 | 5.1 | 5.2 |
| Com. Ex. 1 | 5.8 | 5.8 | 6.1 | 6.5 |
| Com. Ex. 3 | 5.8 | 5.9 | 6.1 | 6.5 |

Table 27 pH change of each preparation under 50°C preservation

| preparation formulation | initial value | 50°C preservation | | | |
|---|---|---|---|---|---|
| | | 1 week later | 2 weeks later | 4 weeks later | 8 weeks later |
| Ex. 1 | 4.8 | 4.8 | 4.9 | 4.9 | 5.0. |

Table continued

| preparation formulation | initial value | 50°C preservation | | | |
|---|---|---|---|---|---|
| | | 1 week later | 2 weeks later | 4 weeks later | 8 weeks later |
| Ex. 5 | 4.9 | 4.9 | 5.0 | 5.0 | 5.1 |
| Ex. 7 | 4.9 | 5.0 | 5.1 | 5.1 | 5.1 |
| Com. Ex. 1 | 5.8 | 5.9 | 6.0 | 6.2 | 6.7 |
| Com. Ex. 3 | 5.8 | 5.8 | 5.9 | 6.1 | 6.5 |

As is clear from the results of the aforementioned Table 16 - Table 27, the germicidal antiseptic composition for dilution of the present invention has high stability against precipitation of chlorhexidine gluconate even when diluted with water having a total hardness of 90-290 mg/L, and is superior in stability as a preparation.

## INDUSTRIAL APPLICABILITY

The germicidal antiseptic composition for dilution of the present invention shows high stability against precipitation of chlorhexidine gluconate even when diluted with water containing various ions, and shows superior preparation stability. Furthermore, the germicidal antiseptic composition for dilution of the present invention is advantageous in that it is free of endocrine disruptor-like substance and causative substance thereof.
This application is based on a patent application No. 2003-116611 filed in Japan, the contents of which are hereby incorporated by reference.

## Claims

**1.** A germicidal antiseptic composition for dilution, which is an aqueous liquid comprising chlorhexidine gluconate as a main ingredient, comprising:

(1) 1-10 w/v% of chlorhexidine gluconate;
(2) 1-10 w/v% of one or more selected from the group consisting of a polyoxyethylene alkyl ether and a polyoxyethylene alkenyl ether, each having an HLB of 10-15 and a congeal point of not more than 35°C;
(3) 0.001-0.5 w/v% of a water-soluble organic monocarboxylic acid having 2 to 6 carbon atoms; and
(4) water.

**2.** The germicidal antiseptic composition for dilution of claim 1, wherein the water-soluble organic monocarboxylic acid having 2 to 6 carbon atoms is one or more selected from the group consisting of acetic acid, gluconic acid and gluconodeltalactone.

**3.** The germicidal antiseptic composition for dilution of claim 1 or 2, further comprising a water-soluble alcohol having 1 to 3 carbon atoms at not more than 10 w/v%.

**4.** The germicidal antiseptic composition for dilution of any of claims 1 - 3, wherein an alkyl chain of polyoxyethylene alkyl ether is an alkyl group having 10 to 14 carbon atoms, and an alkenyl chain of the polyoxyethylene alkenyl ether is an alkenyl group having 14 to 18 carbon atoms.

**5.** The germicidal antiseptic composition for dilution of any of claims 1 - 4, wherein the number of moles of ethylene oxide addition in polyoxyethylene alkyl ether is within the range of 7 to 20, and the number of moles of ethylene oxide addition in polyoxyethylene alkenyl ether is within the range of 7 to 20.

**6.** The germicidal antiseptic composition for dilution of claim 1, which has a chlorhexidine gluconate content within the range of 4-6 w/v%.

**7.** The germicidal antiseptic composition for dilution of claim 1, wherein the content of one or more selected from the group consisting of polyoxyethylene alkyl ether and polyoxyethylene alkenyl ether, each having an HLB of 10-15 and a congeal point of not more than 35°C, is within the range of 2-7 w/v%.

**8.** The germicidal antiseptic composition for dilution of claim 1, wherein the content of the water-soluble organic monocarboxylic acid having 2 to 6 carbon atoms is within the range of 0.01-0.2 w/v%.

**9.** The germicidal antiseptic composition for dilution of any of claims 1 - 8, wherein the polyoxyethylene alkenyl ether is polyoxyethylene oleyl ether.

**10.** A germicidal antiseptic preparation to have a chlorhexidine gluconate content within the range of 0.05-0.5 w/v% by diluting the germicidal antiseptic composition for dilution of claim 1 with water having a total hardness of not more than 300 mg/L or ethanol.

**11.** A method of preventing precipitation of chlorhexidine gluconate under dilution with hard water, which comprises simultaneously adding a water-soluble organic monocarboxylic acid having 2 to 6 carbon atoms and one or more selected from the group consisting of a polyoxyethylene alkyl ether having an HLB of 10-15 and a congeal point of not more than 35°C and a polyoxyethylene alkenyl ether having an HLB of 10-15 and a congeal point of not more than 35°C, to an aqueous liquid containing chlorhexidine gluconate as a main ingredient.

## INTERNATIONAL SEARCH REPORT

International application No. PCT/JP2004/005776

A. CLASSIFICATION OF SUBJECT MATTER
Int.Cl[7] A61K31/155, A61P31/02, 31/04, A01N25/02, 25/22, 25/30, 47/44, A61L2/18

According to International Patent Classification (IPC) or to both national classification and IPC

B. FIELDS SEARCHED

Minimum documentation searched (classification system followed by classification symbols)
Int.Cl[7] A61K31/155, A61P31/02, 31/04, A01N25/02, 25/22, 25/30, 47/44, A61L2/18

Documentation searched other than minimum documentation to the extent that such documents are included in the fields searched

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y | JP 57-9717 A (Nippon Shinyaku Co., Ltd.), 19 January, 1982 (19.01.82), Full text (Family: none) | 1-11 |
| Y | JP 4-49206 A (Maruishi Pharmaceutical Co., Ltd.), 18 February, 1992 (18.02.92), Full text & US 5266598 A | 1-11 |
| Y | JP 10-330799 A (Lion Corp.), 15 December, 1998 (15.12.98), Full text (Family: none) | 1-11 |

[X] Further documents are listed in the continuation of Box C. [ ] See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 07 June, 2004 (07.06.04) | 22 June, 2004 (22.06.04) |
| Name and mailing address of the ISA/ Japanese Patent Office | Authorized officer |
| Facsimile No. | Telephone No. |

Form PCT/ISA/210 (second sheet) (January 2004)

**INTERNATIONAL SEARCH REPORT**

International application No.
PCT/JP2004/005776

C (Continuation). DOCUMENTS CONSIDERED TO BE RELEVANT

| Category* | Citation of document, with indication, where appropriate, of the relevant passages | Relevant to claim No. |
|---|---|---|
| Y | JP 49-116218 A (Masao YOSHIDA), 06 November, 1974 (06.11.74), Full text (Family: none) | 1-11 |
| Y | JP 2000-273004 A (Nicca Chemical Co., Ltd.), 03 October, 2000 (03.10.00), Full text (Family: none) | 3 |
| Y | JP 62-56424 A (Hoechst AG.), 12 March, 1987 (12.03.87), Full text & EP 213601 A1 & US 4920151 A | 3 |
| A | JP 1-104003 A (Imperial Chemical Industries PLC.), 21 April, 1989 (21.04.89), Full text & EP 231080 A1 & FR 2592763 A1 | 1-11 |
| A | JP 2-117998 A (Becton Dickinson and Co.), 02 May, 1990 (02.05.90), Full text & EP 356264 A1 | 1-11 |
| A | JP 9-301858 A (Senju Pharmaceutical Co., Ltd.), 25 November, 1997 (25.11.97), Full text & EP 807440 A2 & US 5908865 A | 1-11 |

Form PCT/ISA/210 (continuation of second sheet) (January 2004)